# EUROPEAN PATENT SPECIFICATION

(11) **EP 2 881 059 B1**
(45) Date of publication and mention of the grant of the patent: **07.06.2023**
(21) Application number: 14196213.4
(22) Date of filing: 04.12.2014
(51) Int. Cl.: A61B 18/14, A61B 5/00, A61B 17/00, A61B 18/00

(54) **Needle catheter utilizing optical spectroscopy for tumor identification and ablation**
Nadelkatheter zur Verwendung optischer Spektroskopie zur Tumoridentifizierung und Ablation
Cathéter à aiguille utilisant une spectroscopie optique pour identification et ablation de tumeurs

(30) Priority: 05.12.2013 US 201314098448
(43) Date of publication of application: 10.06.2015
(73) Proprietor: Biosense Webster (Israel) Ltd., Yokneam 2066717 (IL)
(72) Inventor: Beeckler, Christopher, Brea, CA 92821 (US)
(74) Representative: Carpmaels & Ransford LLP

(56) References cited:
- EP-A2- 1 205 156
- WO-A1-95/05124
- WO-A2-2008/008318
- WO-A2-2013/108194
- US-A- 5 486 161
- US-A1- 2002 133 148
- US-A1- 2006 173 359
- US-A1- 2010 121 163

## Description

### FIELD OF INVENTION

This invention relates to catheters, in particular, pulmonary catheters for ablation and tissue diagnostics.

### BACKGROUND

WO95/05124A1 describes a medical probe device which comprises a torquable catheter having a probe end which includes a stylet. US5486161A describes a medical ablation device comprising a flexible RF electrode wire or tube. US2002/133148A1 describes an ablation device with an elongate probe which carries electrodes for deployment from the distal end of the probe into a bone tumor. WO 95/05124, WO2013/108194 A2, US2006/173359 A1, US2010/121163 A1 and WO2008/008318 A2 refer to additional documents relevant for the present invention.

Radiofrequency (RF) ablation of cardiac and other tissue is a well-known method for creating thermal injury lesions at the tip of an electrode. Radiofrequency current is delivered between a skin (ground) patch and the electrode. Electrical resistance at the electrode-tissue interface results in direct resistive heating of a small area, the size of which depends upon the size of the electrode, electrode tissue contact, and current (density). Further tissue heating results from conduction of heat within the tissue to a larger zone. Tissue heated beyond a threshold of approximately 50-55 degrees C is irreversibly injured (ablated).

Resistive heating is caused by energy absorption due to electrical resistance. Energy absorption is related to the square of current density and inversely with tissue conductivity. Current density varies with conductivity and voltage and inversely with the square of radius from the ablating electrode. Therefore, energy absorption varies with conductivity, the square of applied voltage, and inversely with the fourth power of radius from the electrode. Resistive heating, therefore, is most heavily influenced by radius, and penetrates a very small distance from the ablating electrode. The rest of the lesion is created by thermal conduction from the area of resistive heating. This imposes a limit on the size of ablation lesions that can be delivered from a surface electrode.

Theoretical methods to increase lesion size would include increasing electrode diameter, increasing the area of electrode contact with tissue, increasing tissue conductivity and penetrating the tissue to achieve greater depth and increase the area of contact, and delivering RF until maximal lesion size has been achieved (60-90 seconds for full maturation).

The electrode can be introduced to the tissue of interest directly (for superficial/skin structures), surgically, endoscopically, laparoscopically or using percutaneous transvascular (catheter-based) access. Catheter ablation is a well-described and commonly performed method by which many cardiac arrhythmias are treated. Needle electrodes have been described for percutaneous or endoscopic ablation of solid-organ tumors, lung tumors, and abnormal neurologic structures.

Catheter ablation is sometimes limited by insufficient lesion size. Ablation of tissue from an endovascular approach results not only in heating of tissue, but heating of the electrode. When the electrode reaches critical temperatures, denaturation of blood proteins causes coagulum formation. Impedance can then rise and limit current delivery. Within tissue, overheating can cause steam bubble formation (steam "pops") with risk of uncontrolled tissue destruction or undesirable perforation of bodily structures. In cardiac ablation, clinical success is sometimes hampered by inadequate lesion depth and transverse diameter even when using catheters with active cooling of the tip. Theoretical solutions have included increasing the electrode size (increasing contact surface and increasing convective cooling by blood flow), improving electrode-tissue contact, actively cooling the electrode with fluid infusion, changing the material composition of the electrode to improve current delivery to tissue, and pulsing current delivery to allow intermittent cooling.

Needle electrodes improve contact with tissue and allow deep penetration of current delivery to areas of interest. Ablation may still be hampered by the small surface area of the needle electrode such that heating occurs at low power, and small lesions are created. An improved catheter with needle ablation is disclosed in U.S. Patent No. 8,287,531.

The need and demand for an accurate, non-invasive method for determining biological attributes of tissue are well-documented. Accurate, noninvasive determination of various disease states could allow faster, more convenient screening and diagnosis, allowing more effective treatment. Method and apparatus employing optical spectroscopy for determining tissue attributes are known. For example, U.S. Patent No. 7,623,906 discloses a method and an apparatus for a diffuse reflectance spectroscopy which includes a specular control device that permits a spectroscopic analyzer to receive diffusely reflected light reflected from tissue. U.S. Patent No. 7952719 discloses an optical catheter configuration combining Raman spectroscopy with optical fiber-based low coherence reflectometry. U.S. Patent No. 6,377,841 discloses the use of optical spectrometry for brain tumor demarcation.

Portions of light incident on tissue may be transmitted through the tissue, absorbed as heat, refracted, specularly reflected and diffusely reflected. Light that undergoes multiple refractions within tissue may contain information concerning biological attribute(s) of interest.

Without a catheter that is adapted for both tissue diagnostics and ablation, the use of a separate ablation treatment catheter following tissue diagnosis, including diagnosis by optical spectroscopy, can increase cost and duration of the procedure and pose a risk that the ablation treatment catheter may not be returned to the exact diagnosis location to deliver ablation energy.

Accordingly, it is desirable for a catheter to have at least an electrode needle adapted for both ablation and optical spectroscopy so that tissue diagnostics and ablation can be performed with a single catheter. Such a catheter would provide a "see and treat" device that would reduce procedure time and significantly reduce, if not eliminate, the risk of not returning to the exact biopsy location to deliver ablation treatment.

### SUMMARY OF THE INVENTION

The present invention is defined by the appended claims and addresses the above concerns by providing a catheter that creates enhanced lesions using a needle electrode assembly and employs optical spectroscopy, including transmissive and refractive spectroscopy before, during or after ablation to assess tissue attributes, including malignancy and/or necrosis. The catheter comprises an elongated catheter shaft, a control handle, and a needle electrode assembly that extends through the catheter shaft and the control handle, which can be advanced distally or retracted proximally relative to the catheter shaft for irradiating, penetrating and ablating a target tissue site. The distal end of the needle electrode assembly is adapted to penetrate tissue surface and ablate tissue at a depth below the tissue surface. The distal end of the needle electrode assembly is also adapted to irradiate tissue and collect optical data by providing at least one wave guide, including a fiber optic, that can transmit light energy from the catheter to an optical analyzer, e.g., a spectrometer. In that regard, the wave guide has a distal end generally coterminous with a distal end of the needle to emit light energy onto or into the target tissue. Light energy that has interacted with the target tissue is detected by an additional collector fiber optic. The fiber optics are housed in the needle electrode assembly.

The present invention includes an integrated catheter-based ablation and spectroscopy system having the aforementioned catheter, an RF generator for providing RF energy to the needle electrode assembly, a light source to provide light energy to illuminate target tissue, and an optical analyzer, for example, a spectrometer, to detect and analyze optical data collected by the wave guides. In that regard, it is understood that the spectrometer is any instrument used to probe a property of light as a function of its portion of the electromagnetic spectrum, typically its wavelength, frequency, or energy. The property being measured is often, but not limited to, intensity of light, but other variables like polarization can also be measured. Technically, a spectrometer can function over any range of light, but most operate in a particular region of the electromagnetic spectrum.

The system may also include a patient interface unit and a communication (COM) unit, a processor and a display, where the COM unit provides electronics for ECG, electrogram collection, amplification, filtering and real-time tracing of catheter distal tip and the PIU allows communication with various components of the system, including signal generator, recording devices, etc. The system may include a location pad with magnetic field generators (e.g., coils) to generate magnetic fields within the patient's body. Signals detected by a sensor housed in the catheter in response to the magnetic fields are processed by the processor order to determine the position (location and/or orientation) coordinates of the catheter distal end. Other signals from the catheter, for example, tissue electrical activity and temperature, are also collected by the catheter and transmitted to the COM unit and the processor via the PIU for processing and analysis.

In a more detailed embodiment, the needle electrode assembly has an elongated proximal portion and a shorter distal "needle" portion, and the at least one optical waveguide extends alongside or within lumens of the proximal and distal portions. The distal needle portion can be advanced and retracted through an axial passage formed in a distal tip electrode mounted on a distal end of the catheter body. A ring electrode is mounted proximally of the distal tip electrode. Each of the distal needle portion, the distal tip electrode and the ring electrode is configured within the catheter for separate and independent selective ablation.

According to the invention, the catheter has an emitter fiber optic and a collector fiber optic that extend through the lumens of the needle electrode assembly. Each has a distal end coterminous with the distal end of the distal needle portion, which is beveled to facilitate the distal end piercing and penetrating tissue. The catheter includes a deflection control handle and a needle control handle. A proximal end of the needle electrode assembly is housed in the needle control handle and responsive to a control configured to advance the distal needle portion past a distal end of the catheter body. The needle control handle is also configured to retract the distal needle portion.

An embodiment of the integrated ablation and spectroscopy system of the present invention comprises the aforementioned catheter, an RF generator adapted to provide RF energy to the needle electrode assembly, a light source adapted to provide light energy for the catheter wave guides, and a light analyzer adapted to analyze the light collected by the at least one waveguide. The system may further include a patient interface unit, a communication unit, a processor, and a display, wherein the patient interface unit is adapted to send and receive signals from the RF generator and the communication unit, wherein the communication unit is adapted to send and receive signals from the patient interface unit, wherein the processor is adapted to send and receive signals from the communication unit, and wherein the display is adapted to receive signals from the processor.

### BRIEF DESCRIPTION OF THE DRAWINGS

These and other features and advantages of the present invention will be better understood by reference to the following detailed description when considered in conjunction with the accompanying drawings wherein:
FIG. 1 is a perspective view of a catheter of the present invention, in accordance with one embodiment.
FIG. 2A is a side cross-sectional view of the catheter of FIG. 1, including a junction between a proximal shaft and a distal shaft, along a first diameter.
FIG. 2B is a side cross-sectional view of the catheter of FIG. 1, including a junction between a proximal shaft and a distal shaft, along a second diameter generally perpendicular to the first diameter of FIG. 2A.
FIG. 2C is an end cross-sectional view of the distal shaft of FIGS. 2A and 2B, taken along line C-C.
FIG. 3 is a side cross-sectional view the distal shaft including a needle electrode assembly of the present invention, in accordance with one embodiment.
FIG. 3A is an end cross-sectional view of the distal shaft of FIG. 3, taken along line A-A.
FIG. 3B is an end cross-sectional view of the distal shaft of FIG. 3, taken along line B-B.
FIG. 4 is a side cross-sectional view of a puller wire T-anchor, in accordance with one embodiment.
FIG. 5 is an isometric view of a distal shaft of the catheter of FIG. 1, being deployed in a right atrium.
FIG. 5A is a detailed view of a distal end of the distal shaft of FIG. 5.
FIG. 6 is a side cross-sectional view of the needle control handle of FIG. 1.
FIG. 6A is a detailed view of Area A in FIG. 6.
FIG. 6B is a detailed view of Area B in FIG. 6.
FIG. 7 is a schematic diagram of a system of the present invention, in accordance to one embodiment.
FIG. 7A is a block diagram of the system of FIG. 7.
FIG. 8 is a perspective view of a catheter of the present invention, in accordance with another embodiment.
FIG. 8A is an end cross-sectional view of a distal tip electrode of FIG. 8, taken along line A-A.
FIG. 8B is a side cross-sectional view of the distal tip electrode of FIG. 8A, taken along line B-B.
FIG. 8C is a side cross-sectional view of the distal tip electrode of FIG. 8B, taken along C-C.
FIG. 8D is a side cross-sectional view of a deflectable section of FIG. 8, taken along line D--D.
FIG. 8E is a side cross-sectional view of a deflection control handle of FIG. 8, taken along E-E.
FIG. 8F is a side cross-sectional view of a needle control handle of FIG. 8, taken along F-F.
FIG. 8G is a detailed view of a connector of FIG. 8.
FIG. 8H is an end cross-sectional view of the deflectable section of FIG. 8D, taken along line H-H.
FIG. 9 is an isometric view of a distal shaft of the catheter of FIG. 8, being deployed in a lung.
FIG. 9A is a detailed view of a distal end of the distal shaft of FIG. 9.

### DETAILED DESCRIPTION OF THE INVENTION

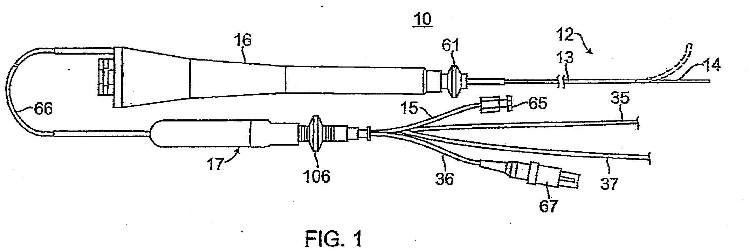

As shown in FIG. 1, the catheter 10 comprises an elongated catheter body 12 having a proximal shaft 13, a distal shaft 14, a deflection control handle 16 attached to the proximal end of the proximal shaft, and a needle control handle 17 attached indirectly to the catheter body 12 proximal of the deflection control handle 16.

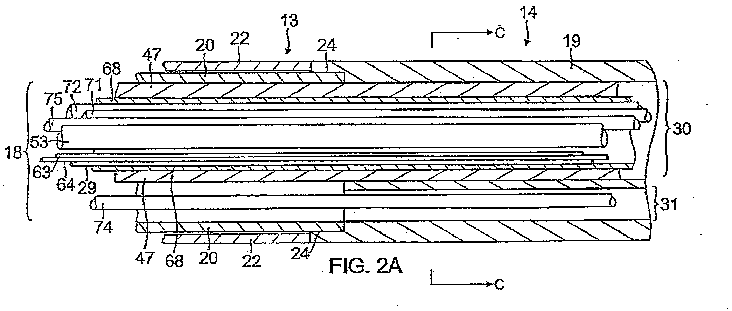

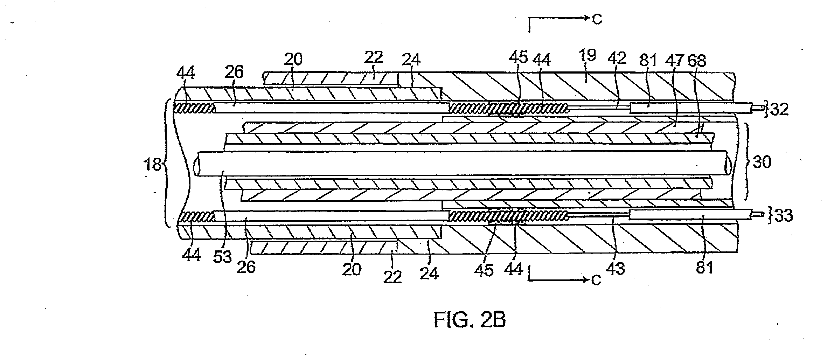

With reference to FIGS. 2A and 2B, the proximal shaft 13 comprises a single, central or axial lumen 18. The proximal shaft 13 is flexible, i.e., bendable, but substantially non-compressible along its length. The proximal shaft 13 may be of any suitable construction and made of any suitable material. A presently preferred construction comprises an outer wall 22 made of polyurethane or nylon. The outer wall 22 comprises an imbedded braided mesh of stainless steel or the like to increase torsional stiffness of the proximal shaft 13 so that, when the deflection control handle 16 is rotated, the distal shaft 14 of the catheter 10 will rotate in a corresponding manner.

The outer diameter of the proximal shaft 13 is not critical, but is preferably no more than about 8 French. Likewise the thickness of the outer wall 22 is not critical. In the depicted embodiment, the inner surface of the outer wall 22 is lined with a stiffening tube 20, which can be made of any suitable material, preferably polyimide. The stiffening tube 20, along with the braided outer wall 22, provides improved torsional stability while at the same time minimizing the wall thickness of the catheter, thus maximizing the diameter of the single lumen. The outer diameter of the stiffening tube 20 is about the same as or slightly smaller than the inner diameter of the outer wall 22.

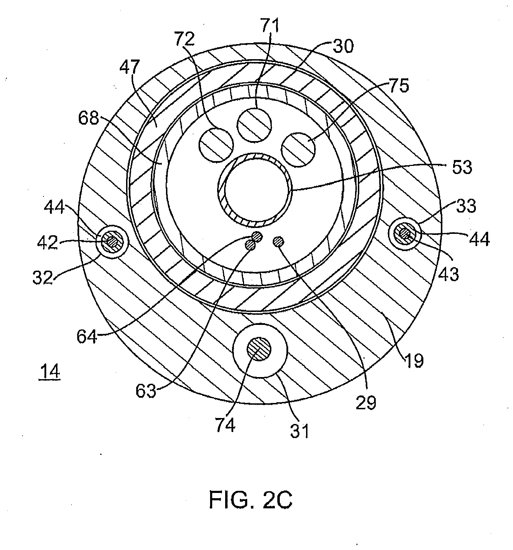

As shown in FIGS. 2A, 2B and 2C, the distal shaft 14 comprises a short section of multi-lumened tubing 19 having, for example, at least three lumens, namely a first lumen 30, a second lumen 31, and a third off-axis puller wire lumen 32 for uni-directional deflection, and a fourth off-axis lumen 33 diametrically opposite of lumen 32 for bidirectional deflection. The tubing 19 is made of a suitable non-toxic material that is preferably more flexible than the proximal shaft 13. A suitable material for the tubing 19 is braided polyurethane, i.e., polyurethane with an embedded mesh of braided stainless steel or the like. The outer diameter of the distal shaft 14, like that of the proximal shaft 13, is preferably no greater than about 8 French.

A suitable means for attaching the proximal shaft 13 to the distal shaft 14 is illustrated in FIGS. 2A and 2B. The proximal end of the distal shaft 14 comprises an inner counter bore 24 that receives the outer surface of the stiffener 20. The distal shaft 14 and proximal shaft 13 are attached by glue or the like. Other methods for attaching the proximal shaft 13 to the distal shaft 14 can be used in accordance with the invention.

The stiffening tube 20 is held in place relative to the outer wall 22 at the proximal shaft 13. In a suitable construction of the proximal shaft 13, a force is applied to the proximal end of the stiffening tube 20, which causes the distal end of the stiffening tube 20 to firmly push against the counter bore 24. While under compression, a first glue joint is made between the stiffening tube 20 and the outer wall 22 by a fast drying glue, e.g. Super Glue.RTM.. Thereafter, a second glue joint is formed between the proximal ends of the stiffening tube 20 and outer wall 22 using a slower drying but stronger glue, e.g., polyurethane.

The depicted catheter includes a mechanism for deflecting the catheter body 12. In the depicted embodiment, the catheter is adapted for bi-directional deflection with a first puller wire 42 extending into the puller wire lumen 32 and a second puller wire 43 extending into the puller wire lumen 33. The puller wires 42 and 43 are anchored at their proximal ends in the deflection control handle 16 and anchored at their distal end at or near a distal end of the distal shaft 14. The puller wires are made of any suitable metal, such as stainless steel or Nitinol, and are preferably coated with Teflon.RTM. or the like. The coating imparts lubricity to the puller wires. Each puller wire preferably has a diameter ranging from about 0.006 to about 0.010 inches (0.15- 0.25 mm).

To effectuate deflection along the distal shaft 14, each puller wire is surrounded by a respective compression coil 44 that extends from the proximal end of the proximal shaft 13 and terminates at or near the proximal end of the distal shaft 14. Each compression coil 44 is made of any suitable metal, preferably stainless steel. The compression coil 44 is tightly wound on itself to provide flexibility, i.e., bending, but to resist compression. The inner diameter of the compression coil 44 is preferably slightly larger than the diameter of the puller wire. For example, when the puller wire has a diameter of about 0.007 inches (0.18 mm), the compression coil preferably has an inner diameter of about 0.008 inches (0.20 mm), The Teflon.RTM. coating on the puller wire allows it to slide freely within the compression coil 44. Along its length, the outer surface of each compression coil 44 is covered by a respective flexible, non-conductive sheath 26 to prevent contact between the compression coils 44 and any other components inside the catheter body 12. The non-conductive sheath 26 may be made of polyimide tubing. Each compression coil 44 is anchored at its proximal end to the proximal end of the stiffening tube 20 in the proximal shaft 13 by glue (not shown). At its distal end, each compression coil is anchored in the respective puller wire lumen 32 and 33 by glue joint 45.

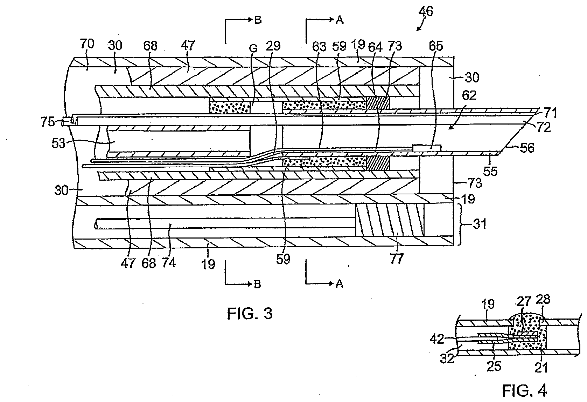

The puller wires are anchored at their distal end to the side of the distal shaft 14, as shown in FIG. 4. In this embodiment, a T-shaped anchor 23 is used for each puller wire. The anchor 23 comprises a short piece of tubular stainless steel 25, e.g., hypodermic stock, which is fitted over the distal end of each puller wire and crimped to fixedly secure it to the puller wire. The distal end of the tubular stainless steel 25 is fixedly attached, e.g., by welding, to a stainless steel cross-piece 27, such as stainless steel ribbon or the like. The cross-piece 27 sits in a notch 28 in a wall of the distal shaft 14 that extends into the second lumen 32. The stainless steel cross-piece 27 is larger than the notch 28 and, therefore, cannot be pulled through the notch. The portion of the notch 28 not filled by the cross-piece 27 is filled with glue 21 or the like, preferably a polyurethane glue, which is harder than the material of the distal shaft 14. Rough edges, if any, of the cross-piece 27 are polished to provide a smooth, continuous surface with the outer surface of the distal shaft 14.

With further reference to FIG. 2B, within the distal shaft 14, the puller wires 42 and 43 extend through a respective protective sheath 81, for example of Teflon.RTM., which prevents the puller wire from cutting into the wall of the distal shaft 14 when the distal shaft is deflected.

Any other suitable technique for anchoring the puller wires 42 and 43 in the distal shaft 14 can also be used. Alternatively, other means for deflecting the distal region can be provided, such as the deflection mechanism described in U.S. Pat. No. 5,537,686.

Longitudinal movement of the puller wires relative to the catheter body 12, which results in deflection of the distal shaft 14, is accomplished by suitable manipulation of the control handle 16 (FIG. 1). Examples of suitable control handles manipulating a single puller wire for unidirectional deflection are disclosed, for example, in U.S. Pat. Nos. Re 34,502, 5,897,529 and 6,575,931. Suitable control handles manipulating at least two puller wires for bidirectional deflection are described in U.S. Pat. Nos. 6,123,699, 6,171,277, and 6,183,463.

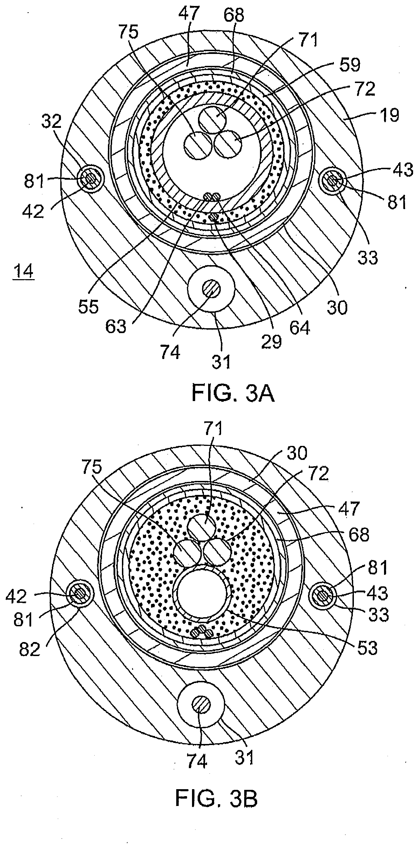

As shown in FIGS. 3, 3A and 3B, a needle electrode assembly 46 is provided. The needle electrode assembly 46 is used to ablate tissue while simultaneously injecting saline or other fluid to conduct the ablation energy and cool the needle electrode, thereby creating a theoretic increase in the effective size of the electrode. The needle electrode assembly 46 is extendable and retractable, and may be moved by manipulation of the needle control handle 17 (FIG. 1), as described further below. FIG. 3 depicts the needle electrode assembly 46 in an extended position relative to the catheter body 12 as it would be to ablate and/or irradiate tissue. The distal end of the needle electrode assembly 46 may be returned or withdrawn into the central lumen 30 to avoid damage to its distal end and/or injury to the patient, particularly during the time that the catheter is advanced through the vasculature or lungs of a patient's body and during the time in which the catheter is removed from the body.

The needle electrode assembly 46 comprises a proximal tubing 53 joined, directly or indirectly, to a generally rigid, electrically-conductive distal tubing or hollow needle 55, as shown in FIG. 3. The generally rigid nature of the needle 55 allows it to pierce tissue in order to increase its effectiveness during ablation. In one embodiment, the needle 55 is formed ofNitinol or stainless steel, According to the invention the needle, as illustrated in FIG. 3, is formed with a beveled edge 56 at the distal tip of the needle electrode assembly 46 to enhance its ability to pierce tissue. The proximal tubing 53 is preferably more flexible than the needle 55 to allow the proximal tubing to bend as necessary with the flexible proximal shaft 13 of the catheter body 12, for instance when the catheter is inserted into the vasculature of the body. The proximal tubing 53 of the needle electrode assembly 46 may be made of polyimide or polyether etherketone (PEEK), but may be made of any other suitable biocompatible material, such as plastic or metal.

The proximal tubing 53 of the needle electrode assembly 46 extends from the needle control handle 17, through the deflection control handle 16, through the proximal shaft 13, and into the lumen 30 of the distal shaft 14. As shown in FIG. 3, the distal end of the proximal tubing 53 and the proximal end of the needle 55 are spaced apart slightly by a discontinuity or gap G so that various components can transition between outside the proximal tubing 53 and inside a lumen of the needle 55. The proximal tubing 53 and needle 55 are mounted, preferably coaxially, within an outer plastic tube 68. The outer plastic tube 68 can be glued or otherwise attached to the proximal tubing 53 and needle 55 to form a single structure that, as described below, is longitudinally movable or slidable relative to the catheter body 12. The outer plastic tube 68 extends through the catheter body 12 with the proximal tubing 53 and protects the needle electrode lead wire 29 and thermocouple wires 63 and 64 which lie and extend between the proximal tubing 53 and outer plastic tube 68, when the needle electrode assembly 46 is moved relative to the catheter body 12. The needle electrode lead wire 29 and thermocouple wires 63 and 64 extend out through a hole (not shown) in the outer plastic tube 68 within the deflection control handle 16 and are attached to appropriate connectors, as noted above.

FIG. 3 shows one arrangement for joining the outer plastic tube 68 to the proximal tubing 53 and needle 55. Specifically, a small piece of plastic tubing 59, for example, polyimide tubing, is placed over and bridges the gap G. The tubing 59 is attached to the proximal and distal tubings by polyurethane glue or the like to form a single fluid passage through which saline or other fluid can pass from the lumen of the proximal tubing 53 to the lumen of the needle 55. The small piece of plastic tubing 59 helps to protect the thermocouple wires 63 and 64 and the needle electrode lead wire 29. A small, e.g., non-conductive, spacer plug 73 is mounted between the distal tubing 55 and the distal end of the outer plastic tube 68, and glued in place. The spacer plug 73 prevents bodily fluid from entering into the distal end of the needle electrode assembly 46.

In one embodiment, the proximal tubing 53 of the needle electrode assembly 46 has an inner diameter of 0.014 inch (0.36 mm) and an outer diameter of 0.016 inch (0.41 mm). The needle 55 has an inner diameter of 0.015 inch (0.38 mm) and an outer diameter of 0.018 inch (0.46 mm) and a length of about 1.0 inch (25.4 mm). Further, the distal tubing 55 extends past the distal end of the distal shaft 14 about 10 mm. The small plastic tubing 59 has an inner diameter of 0.022 inch (0.56 mm) and an outer diameter of 0.024 inches (0.61 mm) the outer plastic tube 68 has an inner diameter of 0.025 inch (0.64 mm) and an outer diameter of 0.035 inch (0.89 mm), and the plastic spacer 73 has an inner diameter of 0.017 inch (0.43 mm) and an outer diameter of 0.024 inch (0.61 mm).

Within the catheter body 12, the needle electrode assembly 46, comprising the proximal tubing 53, needle 55, spacer 73, plastic tubing 59 and outer plastic tube 68, is slidably mounted, preferably coaxially, within a protective tube 47 that lines an inner surface of the lumen 30 and is stationary relative to the catheter body 12. The protective tube 47, which is preferably made of polyimide, serves to prevent the needle electrode assembly 46 from buckling during extension and retraction relative to the catheter body 12. The protective tube 47 additionally serves to provide a fluid-tight seal surrounding the needle electrode assembly 46.

Other needle electrode assembly designs are contemplated within the scope of the invention. For example, the needle electrode assembly can comprise a single electrically-conductive tube, such as a Nitinol tube, that extends from the needle control handle 17 to the distal end of the catheter. Such a design is described in U.S. patent application Ser. No. 09/711,648, entitled "Injection Catheter with Needle Electrode,".

As shown in FIG. 3, a needle electrode lead wire 29 is electrically connected at its distal end to the electrically-conductive distal tubing or needle 55 for supplying radio frequency energy or other suitable ablation energy to the needle. The needle electrode lead wire 29 is soldered, welded or otherwise attached to the outside of the needle 55, but could be attached elsewhere to the needle. The needle electrode lead wire 29 extends generally alongside the proximal tubing 53 and inside of the outer plastic tubing 68, through the lumen 30 of the tubing 19 of the distal shaft 14 and the central lumen 18 of the proximal shaft 13.

A temperature sensor is provided for measuring the temperature of the tissue targeted by the needle electrode assembly 46 before, during or after a procedure. Any conventional temperature sensor, e.g., a thermocouple or thermistor, may be used. In the depicted embodiment, the temperature sensor comprises a thermocouple 62 formed by an enameled wire pair. One wire of the wire pair is a copper wire 63, e.g., a 46 AWG copper wire. The other wire of the wire pair is a constantan wire 64, e.g., a 46 AWG constantan wire. The wires 63 and 64 of the wire pair are electrically isolated from each other except at their distal ends, where they are soldered together, covered with a short piece of plastic tubing 65, e.g., polyimide, and covered with polyurethane. The plastic tubing 65 is then glued or otherwise attached to the inside wall of the needle 55 of the needle electrode assembly 46, as shown in FIG. 3. The wires 63 and 64 extend out the proximal end of the needle 55 and into the gap G to extend generally alongside the lead wire 29 through the lumen 30 of the distal shaft 14, the lumen 18 of the proximal shaft 13 and into the deflection control handle 16. Proximal of the control handle 16, they are attached to an appropriate connector (not shown) connectable to a suitable temperature monitor (not shown). Within the proximal shaft 13 and deflection control handle 16, the lead wire 29 and thermocouple wires 64 and 64 may through a protective tube (not shown), which may be eliminated if desired. In an alternative embodiment, the copper wire 63 of the thermocouple can also be used as the lead wire for the needle electrode assembly 46.

As also shown in FIGS. 3, 3A and 3B, extending along with the needle electrode assembly 46 is at least one wave guide, comprising at least two flexible fiber optics, which extends through the needle control handle 17, the deflection control handle 16, the central lumen 18 of the proximal shaft 13 and the lumen 30 of the distal shaft 14, and into the lumen of the needle 55 of the needle electrode assembly 46 via the gap G. The catheter 10 includes a wave guide bundle 70, for example, at least two individual wave guides. In the illustrated embodiment, the wave guide bundle includes emitter fiber optic 71 and collector fiber optics 72 and 75, although it is understood that the catheter could function suitably with only one emitter wave guide and one collector wave guide. The wave guides extend generally alongside one another and may be bound to each other throughout the catheter body 12. The distal segments of the wave guides inside the needle 55 may be affixed by glue or the like to an inside surface of its lumen. Distal ends of the wave guides are coterminous with the distal end of the needle 55 of the needle electrode assembly 46, i.e. they are similarly beveled as the distal end of the needle 55 for a smooth profile. It is understood that the wave guide bundle 70 may contain any plurality of fiber optics depending on desire and need. For example, the plurality may range between two and six, including one center emitter fiber optic and five surrounding collector fiber optics or any other combinations. Although the wave guides in illustrated embodiment extend along outside of the proximal tubing 53 through the catheter body 12, it is understood that they may extend through the lumen of the proximal tubing, if desired or appropriate.

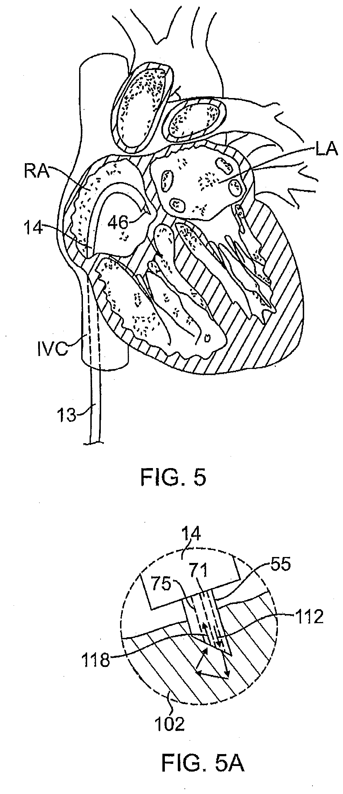

As shown in FIGS. 5 and 5A, the emitter fiber optic 71 is adapted to deliver light energy to the distal end of the needle 55 which has been inserted into target tissue 102 in the right atrium of a patient's heart. Light energy 112 exiting the wave guide may become transmitted light energy, light energy absorbed as heat, and/or refracted light energy 118. Light refracted by tissue back onto the distal ends of the collector fiber optics 72 and 75 is collected and transmitted proximally along the catheter body 12 toward the handles 16 and 17.

In accordance with a feature of the present invention, the wave guide bundle 70 and the needle 55 are arranged at the distal end of the distal shaft 14 such that they are longitudinally parallel, coextensive and/or coaxial for purposes of having their respective distal ends be adapted for at least targeting, contacting and/or piercing a selected target tissue site. That is, by having the distal ends of the wave guides closely aligned with, inside or surrounding the needle 55, it is guaranteed that a biopsy location identified and analyzed by the wave guides will be nearly identical to the location ablated by the needle electrode assembly.

As shown in FIGS. 2A, 2B and 3, within the catheter body 12, the needle electrode assembly 46, electrode lead wire 29, thermocouple wires 63 and 64, and wave guide bundle 70 extending through the outer plastic tubing 68, (collectively referred to herein as the "Outer Plastic Tubing Assembly (OPTA) 68A," are slidably housed within the protective tube 47 that is stationary relative to the catheter body 12. That is, the OPTA 68A is longitudinally movable or slidable relative to the protective tube 47. Within the deflection control handle 16, the protective tube 47 and the OPTA 68A extend into a protective shaft 66 (FIG. 1), which may be made of polyurethane.

Longitudinal movement of the OPTA 68A or at least the needle electrode assembly 46 (and of the wave guide bundle 70 along therewith so they do not break when the needle electrode assembly is extended or retracted) is achieved using the needle control handle 17. The OPTA 68A and the protective tubing 47 extend from the deflection control handle 16 to the needle control handle 17 within the protective shaft 66.

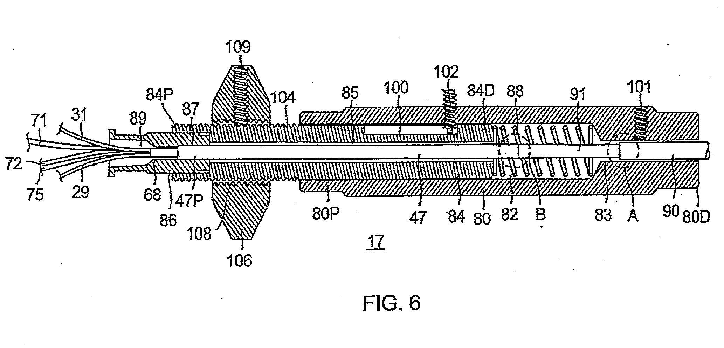

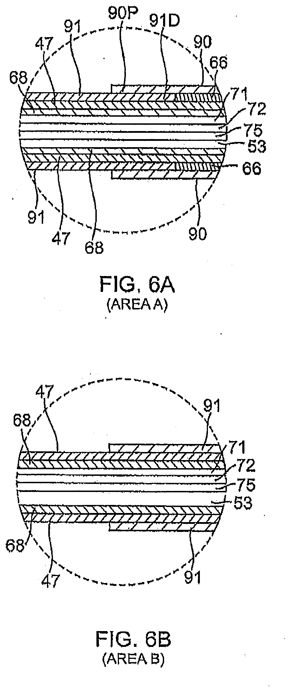

In the illustrated embodiment of FIG. 6, the needle control handle 17 comprises a generally cylindrical outer body 80 having proximal end 80P and distal end 80D, a longitudinal piston chamber 82 extending partially therethrough, and a longitudinal needle passage 83 extending partially therethrough. The piston chamber 82 extends from the proximal end 80P of the outer body 80 partway into the handle 17, but does not extend out the distal end 80D of the outer body. The needle passage 83, which has a diameter less than that of the piston chamber 82, extends from the distal end of the piston chamber to the distal end 80D of the outer body 80.

A piston 84, having proximal end 84P and distal end 84D, is slidably mounted within the piston chamber 82. A proximal fitting 86 is mounted in and fixedly attached to the proximal end 84P of the piston 84. The proximal fitting 86 includes a tubular distal region 87 that extends distally from the main body of the proximal fitting and into the proximal end 84P of the piston. The piston 84 has an axial passage 85 which is coaxial and connects with an axial passage 89 formed in the proximal fitting 86. The OPTA 68A extends through the axial passages 85 and 89, as described in more detail below. A compression spring 88 is mounted within the piston chamber 82 between the distal end 84D of the distal end 84D of the piston 84 and the distal end of the piston chamber 82. The compression spring 88 can either be arranged between the piston 84 and outer body 80, or can have one end in contact with or fixed to the piston 84, while the other end is in contact with or fixed to the distal end 80D of the outer body 80.

From the deflection control handle 16, the OPTA 68A and the protective shaft 66 extend proximally into the distal end of the needle passage 83 of the needle control handle 17. As shown in FIG. 6A, within the needle passage 83, the OPTA 68A and protective shaft 66 extend into a first metal tube 90, which may be made of stainless steel. If desired, the first metal tube 90 could instead be made of a rigid plastic material. The first metal tube 90 is secured to the outer body 80 of the needle control handle 17 by a set screw 101 or any other suitable means. The protective shaft 66 terminates at its proximal end within the first metal tube 90.

A second metal tube 91 is provided, with its distal end 91D received, preferably coaxially, inside proximal end 90P of the first metal tube 90, with the distal end 91D abutting the proximal end of the protective shaft 66. The second metal tube 91 is fixed in place relative to the first metal tube 90 and thus also to the outer body 80 by the set screw 101. The second metal tube 91, like the first metal tube 90, could alternatively be made of a rigid plastic material. As shown in FIG. 6B, the second metal tube 91 terminates at its proximal end 91P which is distal of a proximal end of the compression spring 88 in a neutral state and/or the distal end 84D of the piston 84 in a neutral state. As such, the OPTA 68A extends through the axial passage 85 of the piston 84. The first metal tube 90 serves as a protective sheath to guarantee coaxial alignment over the butt connection of the proximal end of the protective shaft 66 and the distal end of the second metal tube 91.

Proximal end of the OPTA 68A is received in the axial passage 89 of the tubular distal end 87 of the proximal fitting 86. The protective tube 47 terminates at its proximal end in the tubular distal region 87 which exposes proximal end of the OPTA 68A for fixed attachment by glue or the like to an inner surface of the axial passage 89. Thus, the piston 84 and the OPTA 68A are coupled to the proximal fitting 86 for longitudinal movement relative to the second metal tube 91, the first metal tube 90 and the outer body 80. Accordingly, when the piston 84 is moved distally (toward the right in FIG. 6) relative to the outer body 80, the OPTA 68A is moved distally relative to the catheter body 12 thereby advancing the needle electrode assembly 46 for ablation and/or spectroscopy.

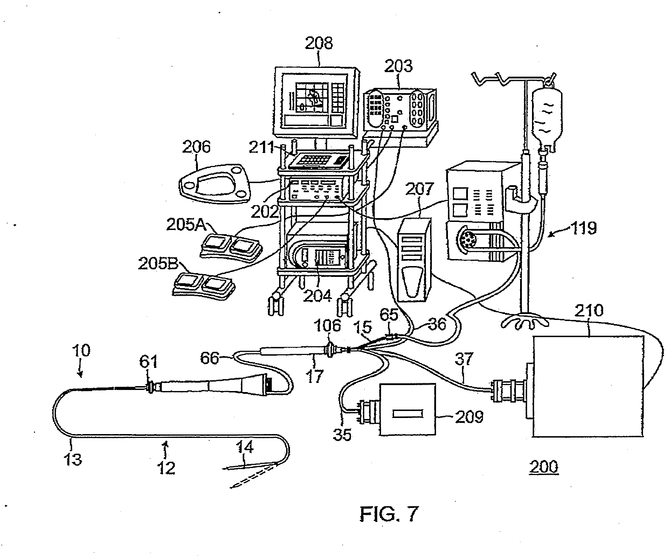

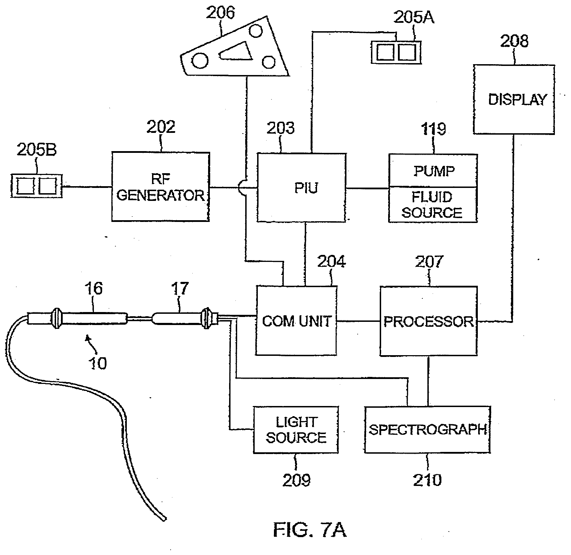

Within the proximal fitting 86, the proximal tubing 53 extends out of the outer plastic tube 68 and into a first protective sheath 15 and is connected to a luer connector 65, which is connected to an irrigation pump or other suitable fluid infusion source 119, as shown in FIG. 7. Similarly, the needle electrode lead wire 29 and the thermocouple wires 63 and 64 extend out of the outer plastic tube 68 and into a second protective sheath 36, as shown in FIG. 7, which is connected to a suitable connector 67, such as a 10-pin electrical connector, for connecting the needle electrode lead wire to a source of ablation energy and the thermocouple wires to a suitable monitoring system. The emitter fiber optic 71 extends out of the outer plastic tube 68 and into a third protective sheath 35, as shown in FIG. 7, which is connected to a suitable light source, which may be a lamp, light emitting diodes (LEDs), or multiple lasers. The collector fiber optics 72 and 75 extend out of the outer plastic tube 88 and into a fourth protective sheath 37, as shown in FIG. 7, which is connected to a suitable light analyzer, e.g., a spectrometer, to process the collected light.

In use, force is applied to the piston 84 to cause distal movement of the piston relative to the outer body 80, which compresses the compression spring 88. This movement causes the OPTA 68A, inclusive of the needle electrode assembly 46 and the wave guide bundle 70, to correspondingly move distally relative to the outer body 80, protective shaft 66, protective tube 47, proximal shaft 13, and distal shaft 14 so that a distal end of the distal tubing 55 of the needle electrode assembly 46 extends outside the distal end of the distal shaft 14. When the force is removed from the piston 84, the compression spring 88 expands and pushes the piston proximally to its original position, thus causing the distal end of the distal tubing 55 of the needle electrode assembly 46, along with the distal ends of the wave guide bundle 70, to retract back into the distal shaft 14. Upon distal movement of the piston 84, the proximal tubing 53 and other plastic tubing 68 move distally into the protective tube 47 to prevent the proximal tubing 53 and the outer plastic tube 68 from buckling within the axial passage 85.

The piston 84 further comprises a longitudinal slot 100 extending along a portion of its outer surface. A securing means 102, such as a set screw, pin, or other locking mechanism, extends radially through the outer body 80 and into the longitudinal slot 100. This design and the set screw limit the distance that the piston 84 can be slid proximally out of the piston chamber 82. When the needle electrode assembly 46 is in the retracted position (as shown in FIG. 6), preferably the securing means 102 is at or near the distal end of the longitudinal slot 100.

The proximal end of the piston 84 has a threaded outer surface 104. A circular thumb control 106 is rotatably mounted on the threaded outer surface 104 at proximal end of the piston 84. The thumb control 106 has a threaded inner surface 108 that interacts with the threaded outer surface 104 of the piston 84 so that the longitudinal position of the thumb control 106 relative to the proximal end 80P of the outer body 80 is adjustable. The thumb control 106 acts as a stop, limiting the distance that the piston 84 can be pushed distally into the piston chamber 82, and thus the distance that the needle electrode assembly 46 can be extended out of the distal end of the catheter body 12. The threaded surfaces of the thumb control 106 and piston 84 allow the thumb control to be moved closer or farther from the proximal end 80P of the outer body 80 so that the extension distance of the needle electrode assembly 46 can be controlled by the physician. A securing means, such as a tension screw 109 is provided in the thumb control 106 to control the tension between the thumb control and piston 84 for locking and releasing the thumb control in a longitudinal position on the proximal end 84P of the piston. As would be recognized by one skilled in the art, the thumb control 106 can be replaced by any other mechanism that can act as a stop for limiting the distance that the piston 84 extends into the piston chamber 82, and it is not necessary, although it is preferred, that the stop be adjustable relative to the piston.

In the depicted embodiment, as shown in FIG. 3, the catheter further includes at least one location sensor 77. The location sensor 77 is used to determine the coordinates of the distal end of the distal shaft 14. Specifically, the location sensor 77 is used to monitor the precise location of the distal end of the catheter in the patient's body. The location sensor 77 is connected to a corresponding sensor cable 74. The sensor cable 74 extends, along with the lead wire 29, through the lumen 31 of the distal shaft 14 (FIG. 2C), and through the proximal shaft 13 within a protective tube (not shown) and then into the deflection control handle 16 and out of the proximal end of the deflection control handle within an umbilical cord (not shown) to a sensor control module (not shown) that houses a circuit board (not shown). Alternatively, the circuit board can be housed within the control handle 16, for example, as described in U.S. Pat. No. 6,024,739. The sensor cable 74 comprises multiple wires encased within a plastic covered sheath. In the sensor control module, the wires of the sensor cable 74 are connected to the circuit board. The circuit board amplifies the signal received from the location sensor 77 and transmits it to a computer in a form understandable by the computer by means of a sensor connector at the proximal end of the sensor control module. Also, because the catheter is designed for single use only, the circuit board may contain an EPROM chip that shuts down the circuit board approximately twenty-four hours after the catheter has been used. This prevents the catheter, or at least the location sensor 77, from being used twice.

The location sensor 77 may be an electromagnetic location sensor. For example, the location sensor 77 may comprise a magnetic-field-responsive coil, as described in U.S. Pat. No. 5,391,199, or a plurality of such coils, as described in International Publication WO 96/05758. The plurality of coils enables the six-dimensional coordinates (i.e. the three positional and the three orientational coordinates) of the location sensor 77 to be determined. Alternatively, any suitable location sensor known in the art may be used, such as electrical, magnetic or acoustic sensors. Suitable location sensors for use with the present invention are also described, for example, in U.S. Pat. Nos. 5,558,091, 5,443,489, 5,480,422, 5,546,951, and 5,568,809, International Publication Nos. WO 95/02995, WO 97/24983, and WO 98/29033.

As shown in FIGS. 7 and 7A, the catheter 10 may be used with an integrated ablation and spectroscopy system 200. In the illustrated embodiment, the system includes an RF generator 202, a patient interface unit 203, a communication (COM) unit 204, a location pad 206, a processor 207, input device (e.g., keyboard) 211, and a display 208. The COM unit 204 provides electronics for ECG, electrogram collection, amplification, filtering and real-time tracing of catheter distal tip. The PIU 203 allows communication with various components of the system 200, including signal generator, recording devices, etc. The location pad 206 includes magnetic field generators (e.g., coils) and is typically positioned under a patient's body to generate magnetic fields within the patient's body. Responsive to these magnetic fields, the location sensor 77 housed in the distal end of the catheter generates electrical signals which are received by the PIU 203 and transmitted to the COM unit 204 and processed by the processor 207 in order to determine the position (location and/or orientation) coordinates of the catheter distal end. The processor 207 uses the coordinates in driving the display 208 to show location and status of the catheter. Other signals from the catheter 10, for example, tissue electrical activity and temperature, are also transmitted to the COM unit 204 and the processor 207 via the PIU 203 for processing and analysis, including 3-D mapping of the patient's heart that is shown on the display 208. This method of position sensing and processing is described in detail, for example, in PCT International Publication WO 96/05768, and is implemented in the CARTO system produced by Biosense Webster Inc. (Diamond Bar, California).

For ablation, the RF generator 202 supplies RF ablation energy to the needle electrode assembly 46 of the catheter 10 via the PIU 203. For spectroscopy, the system 200 further includes a light source 209 which provides incidental light energy to the catheter 10 via the emitter fiber optic 71. Light collected by collector fiber optics 72 and 75 are transmitted to a spectrometer 210 which provides representative signals to the processor 207 which processes the signals to determine various parameters and/or characteristics of the target issue illuminated. The system may include a first foot pedal 205A connected to the PIU 203 to be used for acquiring catheter location points and a second food pedal 205B connected to the RF generator 202 for activating/deactivating the RF generator 202.

To use a catheter of the invention, an electrophysiologist may introduce a guiding sheath and dilator into the patient, as is generally known in the art. A guidewire may also be introduced for a catheter adapted for such use. As shown in FIG. 5, the catheter may be introduced to the right atrium (RA) via the inferior vena cava (IVC). To reach the left atrium (LA), the catheter passes through the septum.

Through the guiding sheath, the entire catheter body 12 can be passed through the patient's vasculature to the desired location. Once the distal end of the catheter reaches the desired location, e.g., the right atrium RA, the guiding sheath is withdrawn to expose the distal shaft 14. The thumb control 61 of the control handle 16 may be manipulated as needed to deflect the distal shaft 14 into position. After the distal end of the catheter body 12 is positioned at a target tissue, the thumb control 106 is depressed to advance the piston 84 of the needle control handle 17. The set screw 102 may be used to releasably lock the piston 84 in selected longitudinal positions relative to the outer body 84 so as to hold the distal end of the needle at particular depths in tissue. As the piston is advanced distally, the OPTA 68A is advanced distally to deploy and expose the needle 55 past the distal end of the catheter into the target tissue. Light energy is transmitted by the emitter fiber optic 71 into the target tissue and light energy scattered back is collected by the collector fiber optics 72 and 77. The collected light is transmitted proximally along the catheter body 12, through the deflection control handle 16 and the needle control handle 17 and to the spectrometer 210 for analysis.

RF energy may be applied to the needle electrode assembly 46 to energize the needle 55 for ablation to create a lesion, including a larger lesion than those created by tissue surface electrode contact. Irrigation fluid may also be provided at the ablation site via the fluid source and pump 119 that provides fluid to be transported through the lumens of the proximal tubing 53 and the needle 55. Advantageously, both spectroscopy and ablation are performed with the use of a single catheter which may remain in situ so that the ablation tissue site is nearly identical to the tissue site of spectroscopy. In that regard, an additional spectroscopic analysis may be performed after ablation to determine whether the ablation was successfully performed. And, if appropriate, an additional ablation procedure may be performed, again with the advantage that the ablation tissue site remains unchanged. Additional spectroscopic analyses and additional ablation procedures may be repeated as many times as needed all at the same tissue site and with the use of a single catheter.

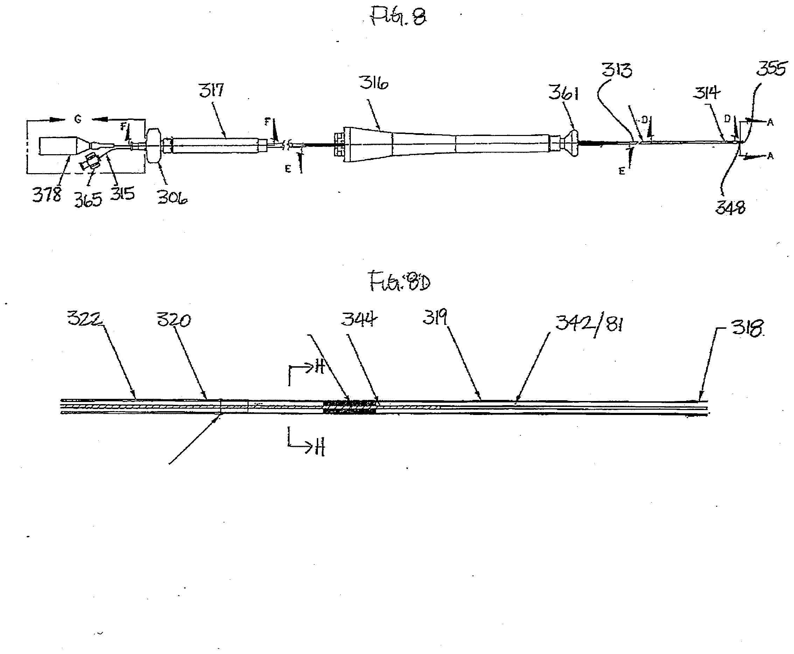

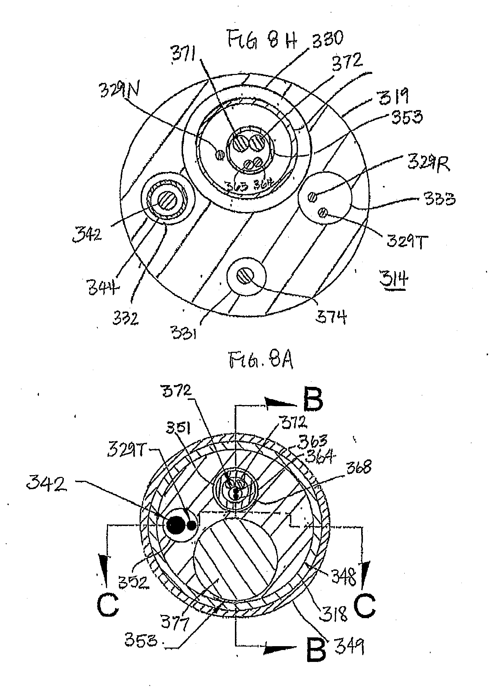

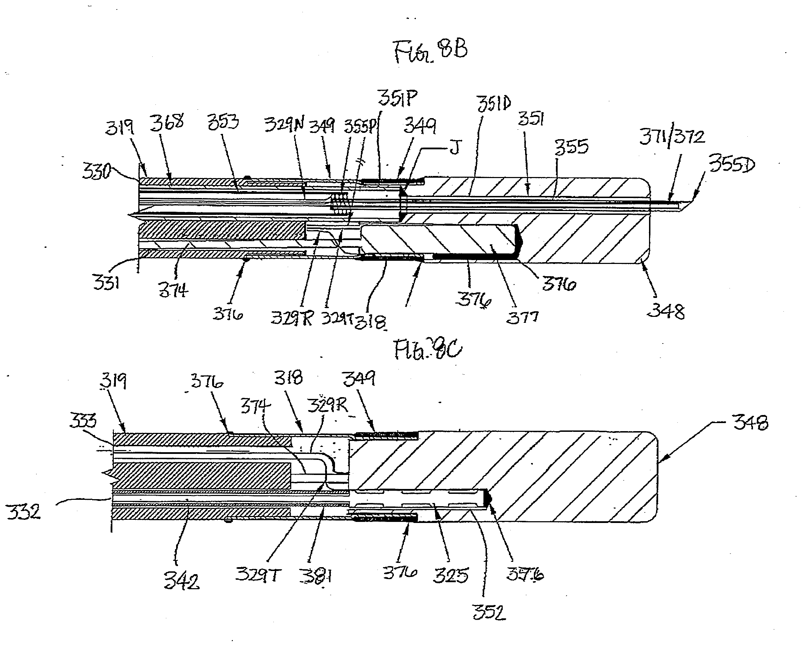

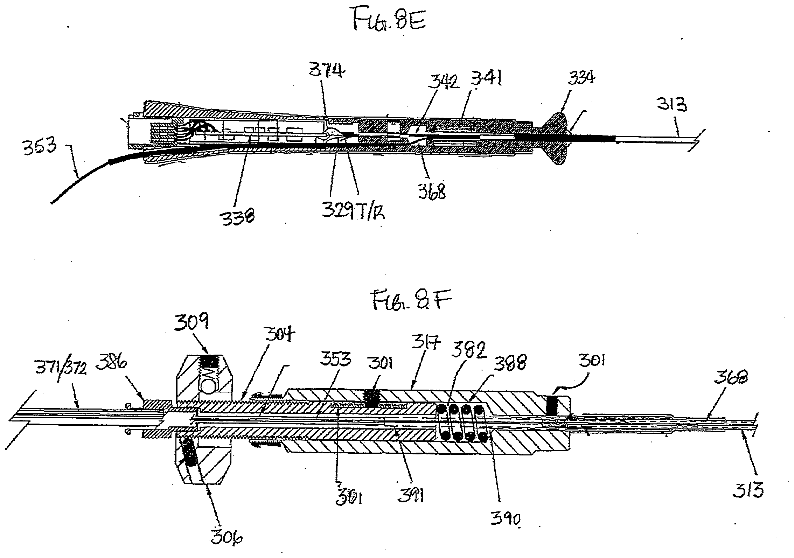

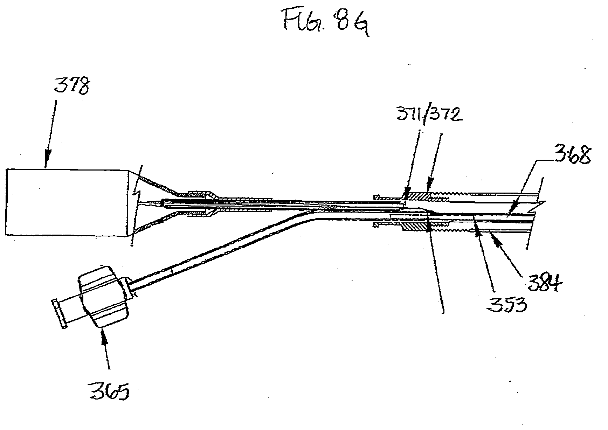

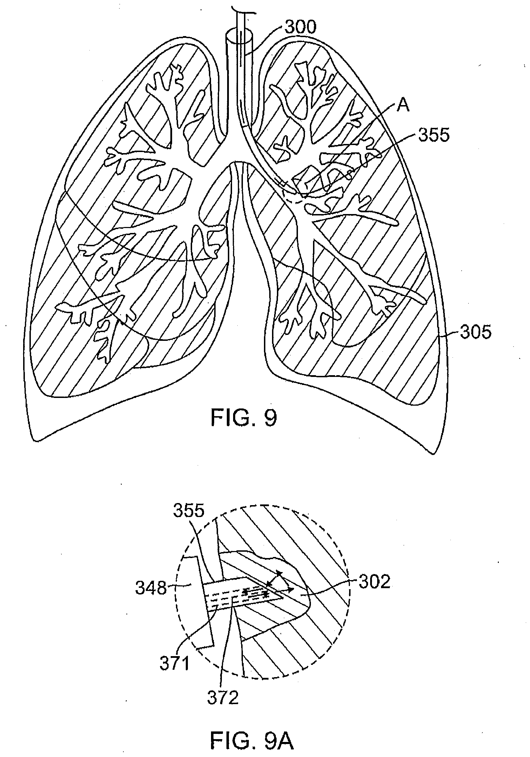

FIGS. 8 and 8A-8H illustrate a catheter 300 of the present invention, in accordance with another embodiment. As shown in FIGS. 9 and 9A, the catheter 300 is adapted for use in a patient's body, including an organ, for example, lungs 305, where it can assess and treat diseased tissue, including a tumor 302. As described below, the catheter 300 has structure similar to the aforementioned catheter 10 in many respects with similar components having similar reference numerals, but there are differences and particular adaptations, including those described below.

The catheter 300 has a proximal shaft 313, a distal shaft 314, a distal tip electrode 348, a ring electrode 349, and a needle electrode assembly 346, which includes a proximal tubing 353 and a distal tubing or "needle" 355. The catheter 300 also includes a deflection control handle 316, a needle control handle 317 distal of the handle 316, and an optical fiber connector 318 distal of the handle 317.

As shown in FIGS. 8D and 8H, the proximal shaft 313 comprises a single, central or axial lumen 318 with an outer wall 322 and a stiffening tube 320. The distal shaft 314 comprises a multi-lumened tubing 319 with at least four lumens 330, 331, 332 and 333. The proximal tubing 353 of the needle electrode assembly 346 extends through the lumen 330. A cable 374 for location sensor 377 extends through the lumen 331. Puller wire 342 extends through the lumen 332. A compression coil 344 surrounding the puller wire extends from the deflection control handle 316 and through the proximal shaft 313 and terminates at its distal end located at a distance distal of the proximal shaft 313, for example, approximately a quarter of the length of the distal shaft 314. Notably, the illustrated embodiment of catheter 300 has unidirectional deflection, so only one puller wire is provided, although it is understood that for bi-directional deflection, a second puller wire and a respective lumen diametrically opposite to the lumen 332 would be provided. Lead wires 329T and 329R for the tip electrode 348 and ring electrode 349, respectively, extend through the lumen 333.

As shown in FIG. 8D, the proximal tubing 353 of the needle electrode assembly 346 houses wave guide bundle (including emitter fiber optic 371 and collector fiber optic 373), lead wire 329 and thermocouple wire pair 363 and 364 for the needle 355. These components extend through a single, center lumen of the proximal tubing 353. Surrounding the proximal tubing 353 along its length is an outer tubing 368 that extends through the needle control handle 317, the deflection control handle 316, the proximal shaft 313, the distal shaft 314 and into the distal tip electrode 348, in which the distal end of the outer tubing 368 is anchored, as described below. Accordingly, in this embodiment, the outer tubing 368 is longitudinally stationary relative to the catheter, and thus not longitudinally moveable with the proximal tubing 353 and needle 355, which can be advanced and withdrawn relative to the catheter body 312 and tip electrode 348. In one embodiment, the outer tubing 368 has an outer layer of polyimide and an inner layer of PTFE, the proximal tubing 353 is constructed of PEEK, and the needle 355 is constructed of nitinol.

At the distal end of the proximal tubing 353 of the needle electrode assembly 346, a proximal end of the needle 355 is received in the lumen of the tubing 353 and anchored therein by a lead wire 329N that extends through the proximal tubing 535. The lead wire 329N is coiled around and soldered to a proximal end 355P of the needle, which is fixed to the distal end of the proximal tubing 353. The fiber optics 370 and 371 and thermocouple wire pair 633 and 634 for the needle 355 also extend through the lumen of the proximal tubing 353 and continue through a single center lumen of the needle 355 where they have distal ends coterminous with a beveled distal end of the needle. Accordingly, the needle electrode assembly 346, comprising the proximal tubing 353, the needle 355, along with the fiber optics 370 and 371, the lead wire 329N and thermocouple wire pair 363 and 364, has longitudinal movement within the outer tubing 368 and relative to the proximal shaft 313 and the distal shaft 314.

With reference to FIGS. 8B and 8C, the tip electrode 348 is mounted on a distal end of the tubing 319 of the distal shaft 314. Between the tip electrode and the tubing 319 is a connector tubing 350, for example, a short section of tubing with a single lumen suitable for housing and allowing reorientation of the wires and/or cables extending from the distal shaft 14 to the tip electrode 348. A proximal end of the connector tubing 350 receives a distal end of the tubing 319 and a distal end of the connector 350 receives a proximal end of the tip electrode 348.

The tip electrode 348 is formed with a longitudinal passage 351 that is axially aligned with the lumen 330 of the distal shaft 314. The passage 351 has a proximal portion 351P with a larger diameter and a distal portion 351D with a smaller diameter. Extending through the proximal portion 351P is the distal end of the wider outer tubing 368 extending from the lumen 330 of the distal shaft 314 which is fixed in the proximal portion 351P. Extending through the outer tubing 368 is the proximal tubing 353 of the needle electrode assembly 346. The proximal tubing 353 has a distal end that is situated between the proximal and distal ends of the connector tubing 318.

The distal portion 351D of the passage 351 receives the needle 355 extending from the proximal tubing 353. As described above, the needle 355 is mounted in the distal end of the proximal tubing 353 and electrically connected by the lead wire 329N. Due to the outer diameter difference between the proximal tubing 353 and the needle 355, a junction J between the distal and proximal portions 351D and 351P of the passage 351 in the tip electrode 348 acts as a distal stop limiting the amount of distal advancement afforded to the needle electrode assembly 346 relative to the tip electrode 348.

The proximal end of the tip electrode 348 is formed with an outer circumferential notch which is received in the distal end of the connector tubing 318. The ring electrode 349 is mounted over the connector tubing 318 in the notch. The ring electrode 249 and the distal end of the connector tubing 318 are bonded to the tip electrode 348 in the notch by glue or the like, for example, polyurethane 376. The lead wire 329R for the ring electrode and the lead wire 329T for the tip electrode both pass from the lumen 333 of the distal shaft 314 and through the lumen of the connector tubing 318. The lead wire 329R is connected to the ring electrode 349 via a hole formed in the connector tubing 318. The lead wire 329T for the tip electrode 348 is soldered in a first blind hole 352 formed in a proximal face of the tip electrode 348. Also anchored in the first blind hole is the distal end of the puller wire 342 which has a crimped short stainless steel tube 323 that is soldered in the hole 354. The proximal face is also formed with a second blind hole 353 to receive the sensor 377 anchored therein. The cable 374 for the biosensor passes from the lumen 331 of the distal shaft 314 and through the lumen of the connector tubing 318.

Accordingly, the catheter 300 has at least three distinguishable electrode elements, namely, the needle 355, the tip electrode 348 and the ring electrode 349, each having its own lead wire for separate and independent selective electrical recording and energization.

FIGS. 8E and 8F illustrate an embodiment of a deflection control handle 316 and a needle control handle 317 suitable for use with the catheter 300. Reference is made to the description herein of the deflection control handle 16 and the needle control handle 17 of FIG 1. Much of the description is applicable to the deflection control handle 316 and the needle control handle 317 of FIGS. 8E and 8F, where similar components are identified by reference numerals with identical last two digits. As for FIG. 8G, an optical fiber connector 378 is shown proximal of the needle control handle 317 and connected thereto, for connecting the fiber optics 371 and 372 to a light source and a spectrometer (FIG. 7). One or more connectors may be provided to define an isolated path for each wave guide.

The preceding description has been presented with reference to presently preferred embodiments of the invention. Workers skilled in the art and technology to which this invention pertains will appreciate that alterations and changes in the described structure may be practiced without meaningfully departing from the scope of the claims. As understood by one of ordinary skill in the art, the drawings are not necessarily to scale. Accordingly, the foregoing description should not be read as pertaining only to the precise structures described and illustrated in the accompanying drawings, but rather should be read consistent with and as support to the following claims which are to have their fullest and fair scope.

## Claims

1. A catheter (10) comprising:
an elongated catheter body (12, 312);
a control handle (16, 17, 316, 317);
a needle electrode assembly (46, 346) extending from the control handle, through the catheter body, the needle electrode assembly having a distal end adapted for penetrating tissue at a target site, the needle electrode assembly being longitudinally movable relative to the catheter body, wherein the needle electrode comprises a distal needle portion (55, 355) which has a lumen and is beveled at its distal end; and
wherein the catheter further comprises at least one optical waveguide adapted to collect light refracted from the tissue at the target site
wherein the at least one optical waveguide comprises an emitter fiber optic (71,371) and a collector fiber optic (73, 373) that extend through the lumen of the needle electrode assembly, each of the emitter and collector fiber optic having a distal end which is positioned in the lumen of the needle portion and is coterminous with the beveled distal end of the distal needle portion.

2. The catheter of claim 1, wherein the emitter fiber optic is adapted to emit light into tissue.

3. The catheter of claim 1, wherein at least a distal end of the emitter fiber optic, the collector fiber optic and at least a distal portion of the needle electrode assembly are coupled for longitudinal movement relative to the catheter body.

4. The catheter of claim 1, wherein the catheter body comprises a proximal shaft (13, 313) and a deflectable distal shaft (14, 314).

5. The catheter of claim 1, further comprising a distal tip electrode, wherein the distal end of the needle electrode assembly is configured for advancement past the distal tip electrode.

6. The catheter of claim 3, further comprising a ring electrode (349).

7. The catheter of claim 1 wherein:
the elongated catheter body has a proximal shaft (13,313) and a distal shaft (14,314);
the needle electrode assembly extends from the control handle, through the proximal shaft and into the distal shaft and has a distal portion having the distal end adapted for penetrating tissue at a target site.

8. The catheter of claim 1 or claim 7, wherein the control handle has a piston (84) adapted to move the needle electrode assembly longitudinally relative to the catheter body/proximal shaft and the distal shaft.

9. The catheter of claim 8, wherein the piston is adapted to advance the distal end of the needle electrode assembly past a distal end of the catheter/distal shaft.

10. The catheter of claim 9, wherein the piston is adapted to retract the distal end of the needle electrode assembly back into the catheter/proximal of the distal end of the distal shaft.

11. A system (200) for ablation and spectroscopy, comprising:
a catheter of claim 1;
an RF generator (202) adapted to provide RF energy to the needle electrode assembly;
a light source (209) adapted to provide light energy into tissue at the target site; and
a spectrometer (210) adapted to analyze the light collected by the at least one waveguide.

12. The system of claim 11, further comprising:
a patient interface unit;
a communication unit (204);
a processor (207); and
a display (208),
wherein the patient interface unit is adapted to send and receive signals from the RF generator, the communication unit,
wherein the communication unit is adapted to send and receive signals from the patient interface unit,
wherein the processor is adapted to send and receive signals from the communication unit,
wherein the display is adapted to receive signals from the processor.

## Patentansprüche

1. Katheter (10), umfassend:
einen länglichen Katheterkörper (12, 312),
einen Steuergriff (16, 17, 316, 317),
eine Nadelelektrodenanordnung (46, 346), die sich von dem Steuergriff durch den Katheterkörper erstreckt, wobei die Nadelelektrodenanordnung ein distales Ende hat, das zum Einstechen in Gewebe an einem Zielort ausgeführt ist, wobei die Nadelelektrodenanordnung in Längsrichtung bezüglich des Katheterkörpers beweglich ist, wobei die Nadelelektrode einen distalen Nadelabschnitt (55, 355) umfasst, der ein Lumen hat und an seinem distalen Ende abgeschrägt ist, und
wobei der Katheter ferner mindestens einen optischen Wellenleiter umfasst, der dazu ausgeführt ist, von dem Gewebe an dem Zielort gebrochenes Licht zu sammeln,
wobei der mindestens eine optische Wellenleiter eine Emitter-Fiberoptik (71, 371) und eine Kollektor-Fiberoptik (73, 373) umfasst, die sich durch das Lumen der Nadelelektrodenanordnung erstrecken, wobei die Emitter- und die Kollektor-Fiberoptik jeweils ein distales Ende haben, das in dem Lumen des Nadelabschnitts positioniert ist und mit dem abgeschrägten distalen Ende des distalen Nadelabschnitts endet.

2. Katheter nach Anspruch 1, wobei die Emitter-Fiberoptik zum Emittieren von Licht in Gewebe ausgeführt ist.

3. Katheter nach Anspruch 1, wobei mindestens ein distales Ende der Emitter-Fiberoptik, die Kollektor-Fiberoptik und mindestens ein distaler Abschnitt der Nadelelektrodenanordnung für eine Längsbewegung bezüglich des Katheterkörpers gekoppelt sind.

4. Katheter nach Anspruch 1, wobei der Katheterkörper einen proximalen Schaft (13, 313) und einen auslenkbaren distalen Schaft (14, 314) umfasst.

5. Katheter nach Anspruch 1, ferner umfassend eine distale Spitzenelektrode, wobei das distale Ende der Nadelelektrodenanordnung zum Vorschieben an der distalen Spitzenelektrode vorbei ausgestaltet ist.

6. Katheter nach Anspruch 3, ferner umfassend eine Ringelektrode (349).

7. Katheter nach Anspruch 1, wobei:
der längliche Katheterkörper einen proximalen Schaft (13, 313) und einen distalen Schaft (14, 314) hat,
sich die Nadelelektrodenanordnung von dem Steuergriff durch den proximalen Schaft und in den distalen Schaft erstreckt und einen distalen Abschnitt hat, der das distale Ende aufweist, das zum Einstechen in Gewebe an einem Zielort ausgeführt ist.

8. Katheter nach Anspruch 1 oder Anspruch 7, wobei der Steuergriff einen Kolben (84) hat, der dazu ausgeführt ist, die Nadelelektrodenanordnung in Längsrichtung bezüglich des Katheterkörpers/proximalen Schafts und des distalen Schafts zu bewegen.

9. Katheter nach Anspruch 8, wobei der Kolben dazu ausgeführt ist, das distale Ende der Nadelelektrodenanordnung an einem distalen Ende des Katheters/distalen Schafts vorbei vorzuschieben.

10. Katheter nach Anspruch 9, wobei der Kolben dazu ausgeführt ist, das distale Ende der Nadelelektrodenanordnung zurück in den Katheter/proximal des distalen Endes des distalen Schafts einzuziehen.

11. System (200) für die Ablation und Spektroskopie, umfassend:
einen Katheter nach Anspruch 1,
einen HF-Generator (202), der dazu ausgeführt ist, HF-Energie für die Nadelelektrodenanordnung bereitzustellen,
eine Lichtquelle (209), die dazu ausgeführt ist, Lichtenergie in Gewebe an dem Zielort bereitzustellen, und
ein Spektrometer (210), das dazu ausgeführt ist, das von dem mindestens einen Wellenleiter gesammelte Licht zu analysieren.

12. System nach Anspruch 11, ferner umfassend:
eine Patientenschnittstelleneinheit,
eine Kommunikationseinheit (204),
einen Prozessor (207) und
eine Anzeige (208),
wobei die Patientenschnittstelleneinheit zum Senden und Empfangen von Signalen von dem HF-Generator, der Kommunikationseinheit, ausgeführt ist,
wobei die Kommunikationseinheit zum Senden und Empfangen von Signalen von der Patientenschnittstelleneinheit ausgeführt ist,
wobei der Prozessor zum Senden und Empfangen von Signalen von der Kommunikationseinheit ausgeführt ist,
wobei die Anzeige zum Empfangen von Signalen von dem Prozessor ausgeführt ist.

## Revendications

1. Cathéter (10) comprenant :
un corps de cathéter allongé (12, 312) ;
une poignée de commande (16, 17, 316, 317) ;
un ensemble d'électrode à aiguille (46, 346) s'étendant depuis la poignée de commande, à travers le corps de cathéter, l'ensemble d'électrode à aiguille ayant une extrémité distale adaptée pour pénétrer dans le tissu au niveau d'un site cible, l'ensemble d'électrode à aiguille étant mobile longitudinalement par rapport au corps de cathéter, l'électrode à aiguille comprenant une partie d'aiguille distale (55, 355) qui a une lumière et est biseautée à son extrémité distale ; et
le cathéter comprenant en outre au moins un guide d'ondes optiques adapté pour collecter la lumière réfractée à partir du tissu au niveau du site cible
l'au moins un guide d'ondes optiques comprenant une fibre optique émettrice (71, 371) et une fibre optique collectrice (73, 373) qui s'étendent à travers la lumière de l'ensemble d'électrode à aiguille, chacune des fibres optiques émettrice et collectrice ayant une extrémité distale qui est positionnée dans la lumière de la partie aiguille et coïncide avec l'extrémité distale biseautée de la partie d'aiguille distale.

2. Cathéter selon la revendication 1, la fibre optique émettrice étant adaptée pour émettre de la lumière dans le tissu.

3. Cathéter selon la revendication 1, au moins une extrémité distale de la fibre optique émettrice, la fibre optique collectrice et au moins une partie distale de l'ensemble d'électrode à aiguille étant couplées pour un mouvement longitudinal par rapport au corps de cathéter.

4. Cathéter selon la revendication 1, le corps de cathéter comprenant une tige proximale (13, 313) et une tige distale déviable (14, 314).

5. Cathéter selon la revendication 1, comprenant en outre une électrode de pointe distale, l'extrémité distale de l'ensemble d'électrode à aiguille étant configurée pour avancer au-delà de l'électrode de pointe distale.

6. Cathéter selon la revendication 3, comprenant en outre une électrode annulaire (349).

7. Cathéter selon la revendication 1,
le corps de cathéter allongé ayant une tige proximale (13, 313) et une tige distale (14, 314) ;
l'ensemble d'électrode à aiguille s'étendant depuis la poignée de commande, à travers la tige proximale et dans la tige distal et ayant une partie distale ayant l'extrémité distale adaptée pour pénétrer dans le tissu au niveau d'un site cible.

8. Cathéter selon la revendication 1 ou la revendication 7, la poignée de commande ayant un piston (84) adapté pour déplacer l'ensemble d'électrode à aiguille longitudinalement par rapport au corps de cathéter/à la tige proximale et à la tige distale.

9. Cathéter selon la revendication 8, le piston étant adapté pour faire avancer l'extrémité distale de l'ensemble d'électrode à aiguille au-delà d'une extrémité distale du cathéter/de la tige distale.

10. Cathéter selon la revendication 9, le piston étant adapté pour rétracter l'extrémité distale de l'ensemble d'électrode à aiguille dans le cathéter/à proximité de l'extrémité distale de la tige distale.

11. Système (200) d'ablation et de spectroscopie, comprenant :
un cathéter selon la revendication 1 ;
un générateur RF (202) adapté pour fournir une énergie RF à l'ensemble d'électrode à aiguille ;
une source de lumière (209) adaptée pour fournir une énergie lumineuse dans le tissu au niveau du site cible ; et
un spectromètre (210) adapté pour analyser la lumière collectée par l'au moins un guide d'ondes.

12. Système selon la revendication 11, comprenant en outre :
une unité d'interface patient ;
une unité de communication (204) ;
un processeur (207) ; et
un dispositif d'affichage (208),
l'unité d'interface patient étant adaptée pour envoyer et recevoir des signaux du générateur RF, de l'unité de communication,
l'unité de communication étant adaptée pour envoyer et recevoir des signaux de l'unité d'interface patient,
le processeur étant adapté pour envoyer et recevoir des signaux de l'unité de communication
le dispositif d'affichage étant adapté pour recevoir des signaux du processeur.
